# EUROPEAN PATENT SPECIFICATION

(11) **EP 2 085 050 B1**
(45) Date of publication and mention of the grant of the patent: **22.09.2010**
(21) Application number: 09250158.4
(22) Date of filing: 21.01.2009
(51) Int. Cl.: A61F 2/04, A61F 2/90

(54) **Biodegradable double stent**
Biologisch abbaubarer Doppelstent
Endoprothèse double biodégradable

(30) Priority: 29.01.2008 KR 20080009090; 21.07.2008 KR 20080070413; 23.10.2008 KR 20080104293
(43) Date of publication of application: 05.08.2009
(73) Proprietor: Taewoong Medical Co., Ltd., Kimpo-si Kyonggi do 415-873 (KR); Shin, Kyong-Min, Seoul (KR)
(72) Inventor: SHIN, Kyong-Min, Seoul (KR); NAM, Jeung-Hee, Kyunggi-do (KR)
(74) Representative: Forrester, Simon Joseph

(56) References cited:
- EP-A- 0 923 912
- WO-A-95/09586
- US-A1- 2004 167 605
- US-B1- 6 221 099

## Description

### BACKGROUND OF THE INVENTION

### Field of the Invention

The present invention relates, in general, to a stent that is used for a patient who does not suffer from a malignant tumor but rather from chronic obstructive pulmonary disease (COPD) in which microtubules such as terminal tracts, bronchioles, etc. of the lungs are blocked, or a patient who suffers from tract stenosis that temporarily manifests itself after various organs such as a biliary tract, an esophagus, an airway and a ureter as well as the surroundings thereof are operated. More particularly, the present invention relates to a biodegradable double stent, in which a biodegradable stent having a hollow cylindrical body woven out of a separate wire made of biodegradable polymer so as to have a plurality of rhombic spaces is fixed to an intermediate portion of a primary stent having a cylindrical body, and in which the biodegradable stent maintains a firmly supported state by pressurizing the inner wall of an organ when inserted into the organ, and then is gradually degraded away by bodily fluids while a lesion area is being cured, thereby enabling the primary stent to be easily removed from the organ.

### Description of the Related Art

Generally, in order to keep narrowed excretory passages expanded at places where microtubules such as terminal tracts, bronchioles, etc. of the lungs are blocked due to chronic obstructive pulmonary disease (COPD) rather than a malignant tumor, and in order to widen the excretory passages of various organs, such as such as a biliary tract, an esophagus, an airway and a ureter, which are narrowed by lesions or which are temporarily narrowed when surroundings of the organs are resected and sutured, various stents are used.

However, the stents administered to the microtubules of the lungs and particularly to narrowed excretory passages, have a problem because they deviate from the narrowed excretory passages without maintaining a firmly supported state due to inspiratory or expiratory pressure caused by respiration.

In order to solve this problem, a stent administrated into a narrowed tract of a lung, a biliary tract, a large intestine, a small intestine, an esophagus, or the like has been disclosed, wherein curved catch ridges protrude from an outer circumference of the middle of the stent.

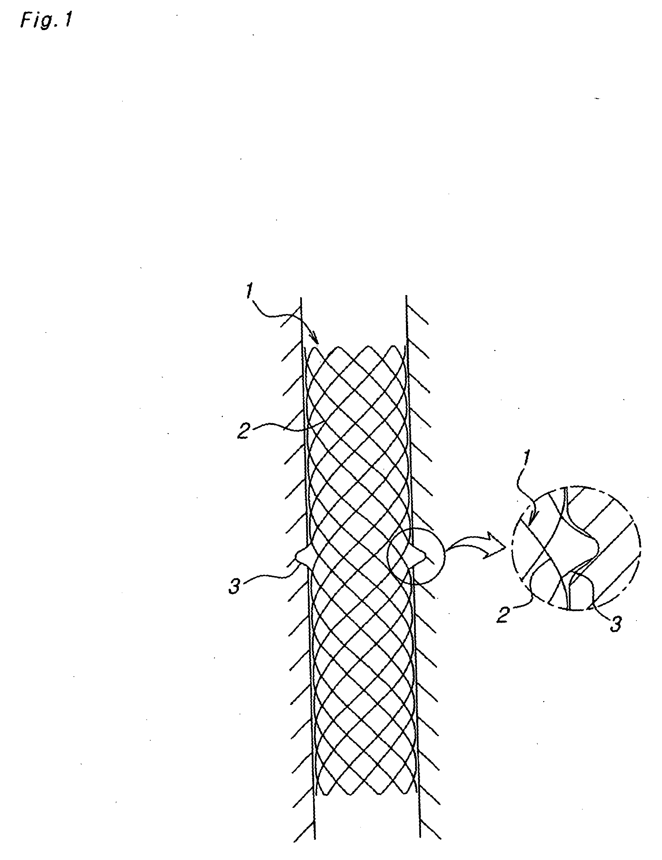

In detail, as illustrated in FIG. 1, when the stent 1 is fabricated using a wire 1, curved catch ridges 3 protrude from an outer circumference of the middle of the stent.

This stent is administrated into the narrowed passage of the lung, the biliary tract, the large intestine, the small intestine, the esophagus, or the like, and thus the curved catch ridges 3 formed on the outer circumference of the middle of the stent offer close contact support with an inner wall of the narrowed passage.

However, the conventional stent is configured such that the curved catch ridges 3 formed on the outer circumference of the middle thereof offer close contact support with an inner wall of the narrowed passage. As such, the stent does not firmly resist external pressure or force.

In other words, the stent is not firmly maintained in the narrowed tract, because of the inspiratory or expiratory pressure caused by respiration of the lung or the force of substances such as bile of the biliary tract or food passing through the large intestine, the small intestine or the esophagus.

After surroundings of the organ are excised and then sutured, the passage of the organ is temporarily narrowed during the healing process. Thus, this stent is used to expand the narrowed passage, and then must be removed after a lesion area has completely cured. At the time of removal, the catch ridges greatly inhibit the stent from being removed from the organ.

EP 0923912 discloses a stent-graft with a bioabsorbable structure and a permanent graft for luminal support and treatment of arterial fistulas, occlusive disease, and aneurysms. The bioabsorbable structure is formed from braided filaments of materials such as PLA, PLLA, PDLA, and PGA and the graft is formed from materials such as PET, ePTFE, PCU or PU. After implantation, the bioabsorbable stent bioabsorbs over time and the generally compliant permanent graft and natural tissue remain in the vessel at the treatment site and form a composite vessel wall.

### SUMMARY OF THE INVENTION

The present invention is defined in the attached claim to which reference should now be made. Further, preferred features may be found in the sub-claims appended thereto.

Accordingly, the present invention has been made keeping in mind the above problems occurring in the related art, and embodiments of the present invention provide a biodegradable double stent, which is firmly maintained without a change in position such as sliding after being administered into a lesion area, and which is allowed to be easily removed after the lesion area has cured.

According to one aspect of the present invention, there is provided a double-tube type biliary stent, which includes a primary stent having a hollow cylindrical body that has a plurality of rhombic spaces formed by weaving an alloy wire made of superelastic shape memory alloy or stainless steel so as to be crossed and that is inserted into and expands an excretory passage of an organ, and a biodegradable stent having a hollow cylindrical body that has a plurality of rhombic spaces formed by weaving a separate wire made of biodegradable polymer so that they criss-cross each other and that has a curved portion curved outwards at an intermediate portion thereof.

According to embodiments of the present invention, the biodegradable double stent is used for temporary administration followed by removal after a predetermined time period has elapsed.

At this time, when the biodegradable double stent is administrated into the passage of an organ, the biodegradable stent made of biodegradable polymer supports an inner wall of the passage of the organ by exerting a predetermined pressure, so that it can be firmly maintained in the passage of the organ.

The biodegradable double stent is firmly maintained without changes in its position caused by sliding and the like, after being administered into the passage of the organ. After a predetermined time period has elapsed, the biodegradable stent is gradually degraded away by bodily fluids when a lesion area of the passage of the organ has completely cured.

This degradation of the biodegradable stent can remove the primary stent from the passage of the organ with ease.

### BRIEF DESCRIPTION OF THE DRAWINGS

The above and other objects, features and advantages of the present invention will be more clearly understood from the following detailed description when taken in conjunction with the accompanying drawings, in which:
FIG. 1 is a front view illustrating a conventional biliary stent;
FIG. 2 is a disassembled perspective view illustrating a biodegradable double stent according to an exemplary embodiment of the present invention;
FIG. 3 is an assembled perspective view of the biodegradable double stent of FIG. 2;
FIGS. 4A and 4B are sectional views of the biodegradable double stent of FIG. 2;
FIGS 5A through 10 illustrate a biodegradable double stent according to another embodiment of the present invention;
FIGS. 11 through 14 illustrate the operation of a biodegradable double stent according to another embodiment of the present invention; and
FIGS 15 through 22 illustrate a biodegradable double stent according to another embodiment of the present invention.

### DETAILED DESCRIPTION OF THE INVENTION

Reference will now be made in greater detail to an exemplary embodiment of the invention, an example of which is illustrated in the accompanying drawings. Wherever possible, the same reference numerals will be used throughout the drawings and the description to refer to the same or like parts.

An exemplary embodiment of the present invention is characterized in that a biodegradable stent having a hollow cylindrical body that has a plurality of rhombic spaces formed by weaving a separate wire made of biodegradable polymer is fixed to an intermediate portion of a primary stent having a hollow cylindrical body that has a plurality of rhombic spaces formed by weaving a superelastic shape memory alloy wire or a stainless steel wire so as to be criss-crossed.

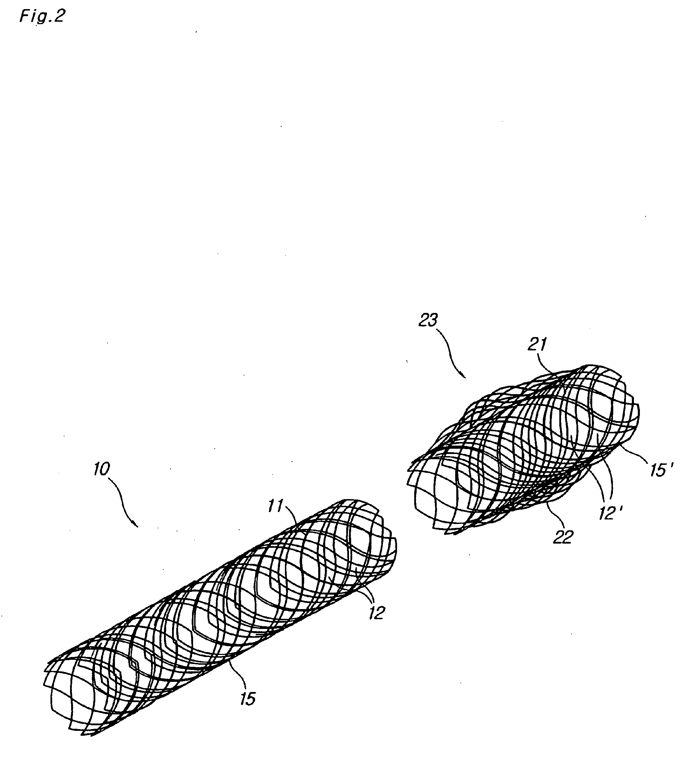

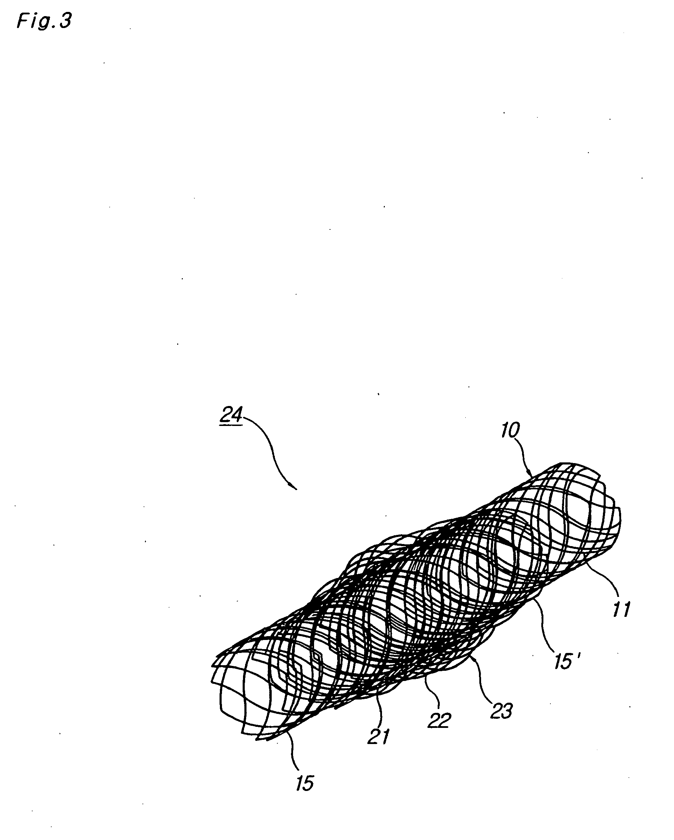

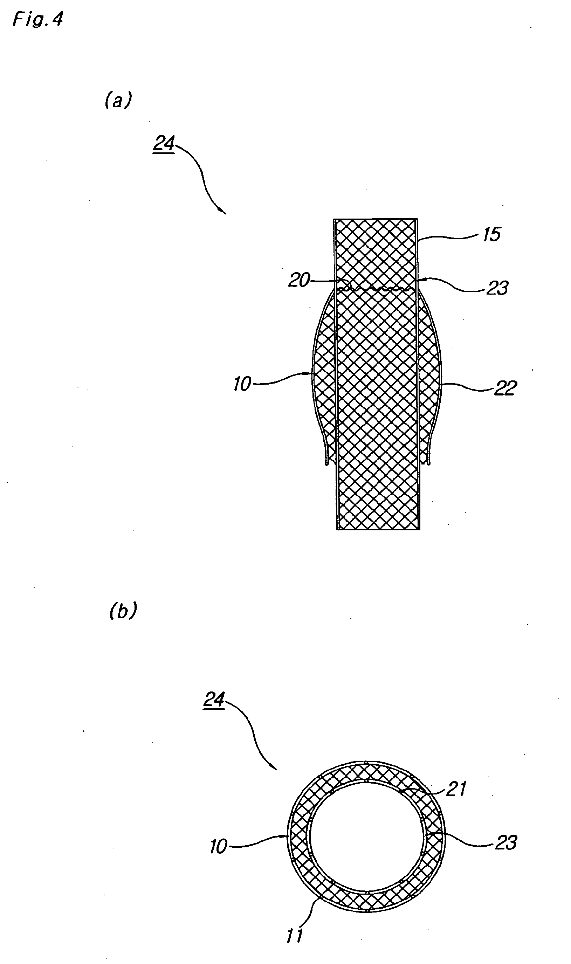

In detail, as illustrated in FIGS. 2 through 4B, a biodegradable double stent includes: a primary stent 10 having a hollow cylindrical body 15 that has a plurality of rhombic spaces 12 formed by weaving an alloy wire 11 made of superelastic shape memory alloy or stainless steel so as to be criss-crossed and that is inserted into and expands the excretory passage of an organ; and a biodegradable stent 23 having a hollow cylindrical body 15' that has a plurality of rhombic spaces 12' formed by weaving a separate wire 21 made of biodegradable polymer so as to be criss-crossed and that has a curved portion 22 curved outwards at an intermediate portion thereof. Here, the biodegradable stent 23 is located on an outer circumference of the primary stent, and is connected to the primary stent 10 at one of opposite ends thereof using a connecting thread or a connecting wire 20.

The biodegradable stent 23 is made of a separate wire 21 of biodegradable polymer. The biodegradable polymer includes one selected from poly L-lactic acid (PLLA), poly lactic acid (PLA), polyglycolic acid (PGA), poly glycolide-co-L-lactide acid (PGLA), polydioxanone (PDO), poly glycolide-co-caprolactone (PGCL), and so on.

However, it should be noted that the biodegradable polymer for the biodegradable stent 23 can use any material innocuous to the human body because it is configured to facilitate removal of the primary stent 10 after the predetermined time period which it takes to be gradually degraded away by bodily fluids has elapsed.

Further, herein, the biodegradable stent 23 is installed on an outer circumference of the primary stent 10. However, the biodegradable stent 23 may be installed on a part, for instance, a middle portion or one end, of the outer circumference of the primary stent 10 if necessary.

Further, the biodegradable stent 23 may be connected to the primary stent 10 at either end thereof in the state in which it is installed on the outer circumference of the primary stent 10. If necessary, the biodegradable stent 23 may be connected to the primary stent 10 at opposite ends thereof in the state in which it is installed on the outer circumference of the primary stent 10.

In this manner, the biodegradable stent 23 can be variously modified.

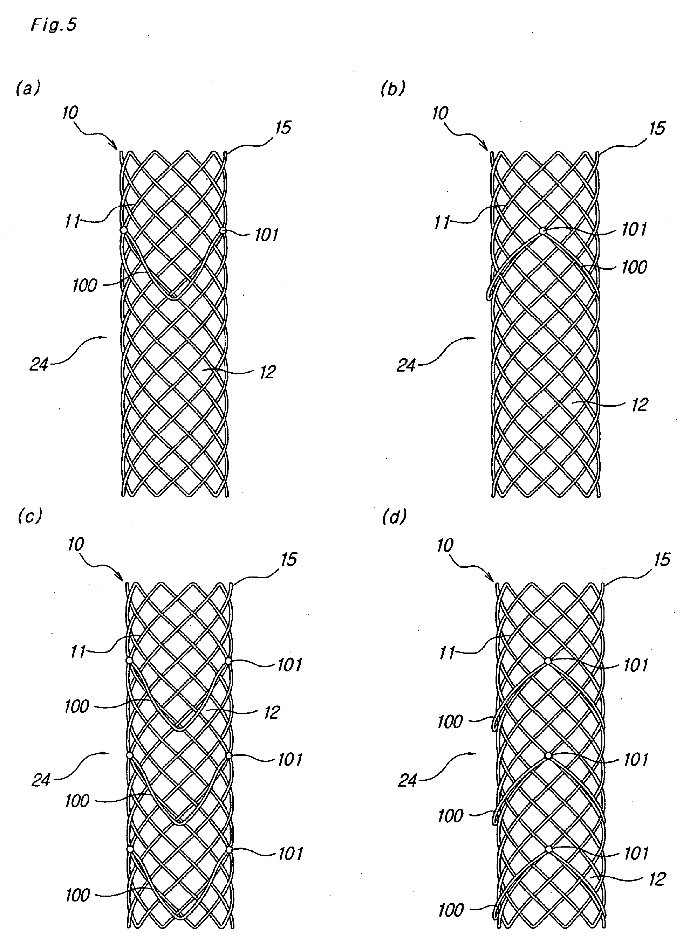

As illustrated in FIGS. 5A, 5B, 5C and 5D, the biodegradable double stent 24 is configured so that the biodegradable stent 23 is installed on the outer circumference of the primary stent 10, wherein the biodegradable stent 23 is formed as a hook-shaped stent 100 curved in a downward direction and is fastened to the primary stent 10 at opposite ends thereof using a fasting means 101.

At this time, at least one hook-shaped stent 100 is installed on the outer circumference of the primary stent 10. In detail, the two hook-shaped stents 100 can be installed so as to be opposite to each other in a diametrical direction. In addition, the numerous hook-shaped stents 100 can be installed in one or more rows at regular intervals in a longitudinal direction.

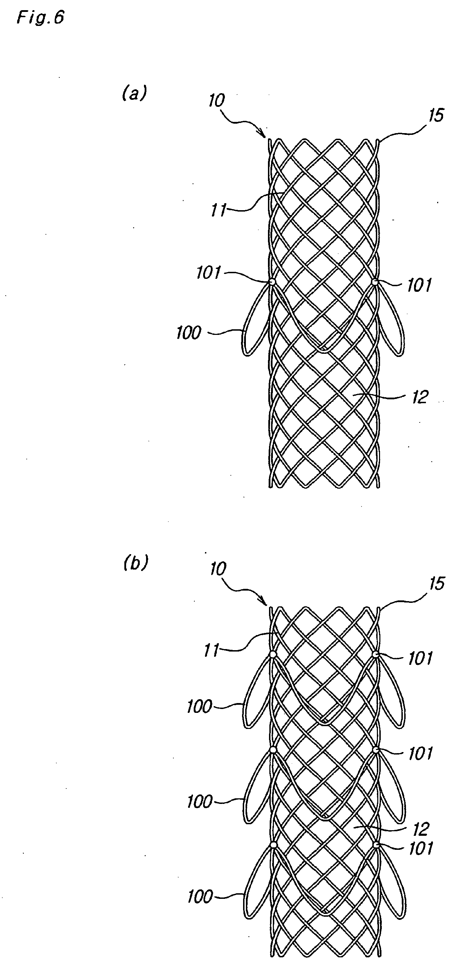

Further, as illustrated in FIGS. 6A and 6B, the several hook-shaped stents 100 can be installed so as to be opposite to each other in a diametrical direction of the primary stent 10. In addition, the numerous hook-shaped stents 100 can be installed in one or more rows at regular intervals in a longitudinal direction of the primary stent 10.

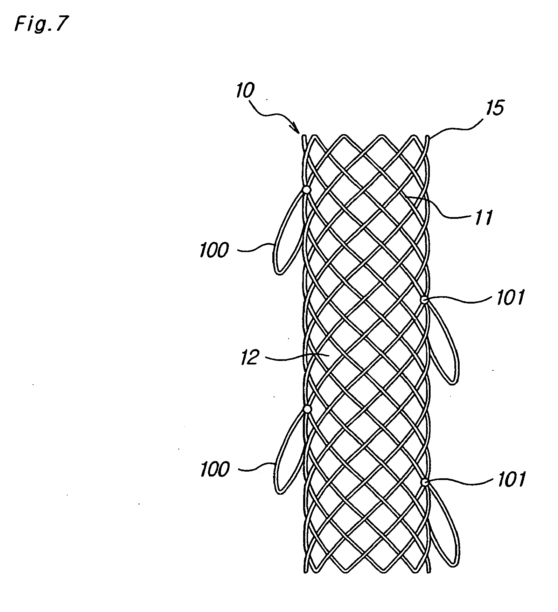

As illustrated in FIG. 7, the numerous hook-shaped stents 100 can be installed in a zigzag shape in a longitudinal direction of the primary stent 10.

At this time, each hook-shaped stent 100 is made of a separate wire 21 of biodegradable polymer. The biodegradable polymer includes one selected from poly L-lactic acid (PLLA), poly lactic acid (PLA), polyglycolic acid (PGA), poly glycolide-co-L-lactide acid (PGLA), polydioxanone (PDO), poly glycolide-co-caprolactone (PGCL) and so on.

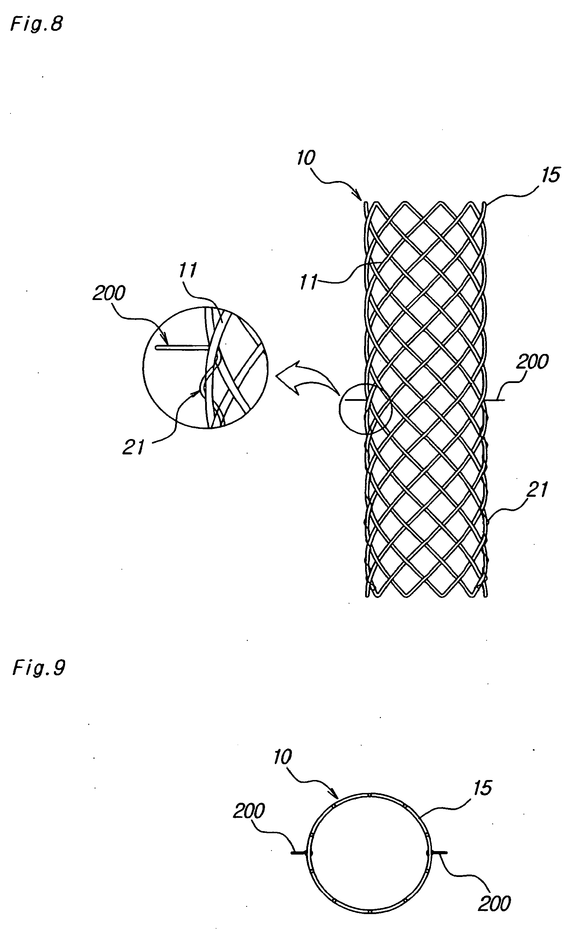

Further, the biodegradable stent 23 can be implemented as illustrated in FIGS. 8 and 9.

As described above, the biodegradable stent 23 is made of a separate wire 21 of biodegradable polymer, and is configured so that one end thereof is formed as a pressurization end 200 protruding outwardly from the outer circumference of the primary stent 10 and that the rest thereof is interwoven with the primary stent 10.

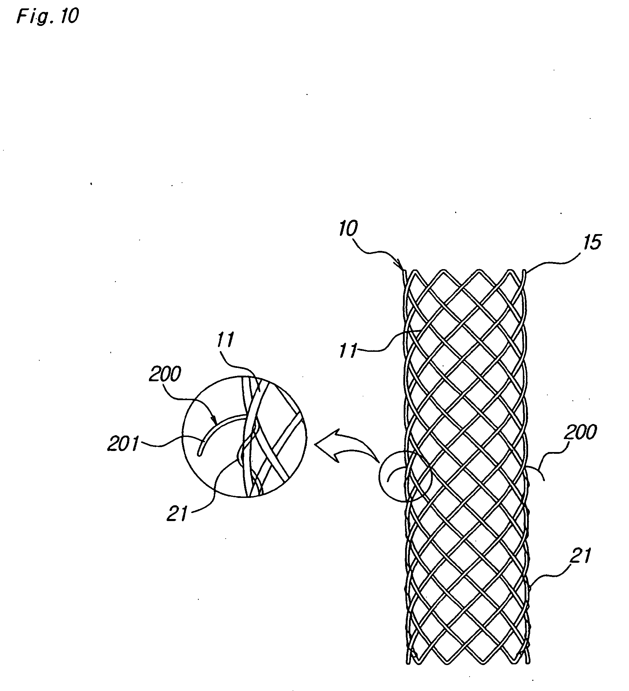

This structure is preferably formed on opposite sides of the primary stent 10. In this case, the pressurization end 200 of the biodegradable stent 23 is formed in a linear shape as illustrated in FIG. 8, or otherwise in a hook shape as illustrated in FIG. 10.

Now, the operation and effects of the biodegradable double stent having the aforementioned configuration will be described below.

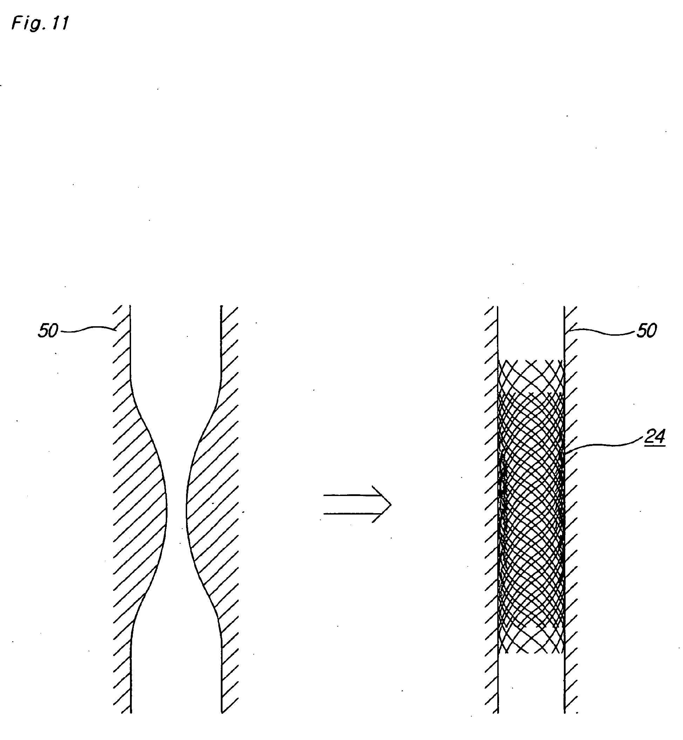

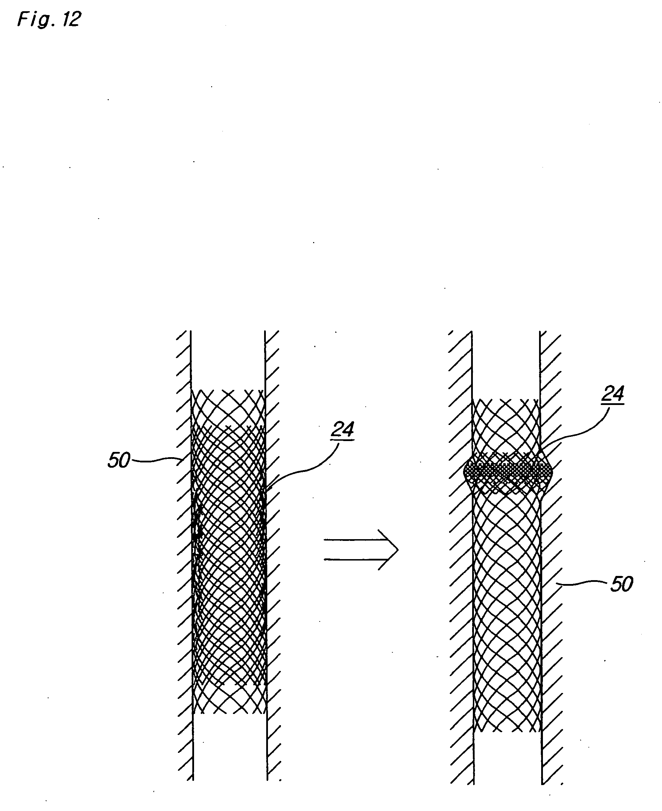

It should be noted that the description of FIGS. 11 and 12 is merely made for the illustrative purpose but not for the purpose of limiting the invention to application in a lung 50.

First, the biodegradable double stent is administrated on the desired area of an organ using separate surgical tools.

When this administration is performed, the primary stent 10 expands the excretory passage of a lesion area for its original purpose, and particularly is inserted into narrowed excretory passages of various organs or microtubules such as terminal tracts, bronchioles, etc. of the lungs of a person who does not suffer from a malignant tumor, or temporarily narrowed excretory passages occurring when surroundings of such organs are excised and sutured using insertion tools, and thereby expands the narrowed excretory passages.

At this time, the biodegradable stent 23 located outside the primary stent 10 supports and pressurizes an inner wall of the excretory passage of the administrated organ, and thus is caught on the inner wall of the excretory passage. In detail, the curved portion 22 of the biodegradable stent 23, an intermediate portion of which protrudes in an outward direction, supports and pressurizes the inner wall of the excretory passage of the organ where it was administered, and thus is caught on the inner wall of the excretory passage.

This catching action allows the biodegradable double stent to be firmly maintained in an inner wall of the excretory passage without deviation from the inner wall of the excretory passage.

More specifically, the biodegradable double stent does not deviate from the administered position, because the biodegradable stent 23 pressurizes the inner wall of the excretory passage although the primary stent 10 be forcibly pushed in a downward direction by excessive force caused by endocrine secretion or food passing through the excretory passage of the organ.

Further, as illustrated in FIG. 12, although the primary stent 10 attempts to slide downwards due to this external force, the primary stent 10 stands against the external force in interaction with the biodegradable stent 23 because the primary stent 10 is coupled with the biodegradable stent 23, and because the biodegradable stent 23 pressurizes the inner wall of the excretory passage of the organ. Thus, the biodegradable stent 23 resists against the sliding due to the pressurizing force of the biodegradable stent 23 which is applied to the inner wall of the excretory passage of the organ, and thus the biodegradable stent 23 is contracted to undergo an increase in its diameter in proportion to such contraction, so that the biodegradable stent 23 further pressurizes the inner wall of the excretory passage of the organ in an outward direction.

Thus, although the primary stent 10 is pushed outwards by such temporary external force, the primary stent 10 is temporarily pushed without deviation from the administered position, and then is returned to its original position again thanks to the supporting force of the biodegradable stent 23 when the external force is removed.

In particular, when the biodegradable double stent 24 is administered to the lesion area of a pulmonary disease, the aforementioned action allows the primary stent 10 to be firmly maintained without deviation from the administered position because the curved portion 223 of the biodegradable stent 23 pressurizes the inner wall of the narrowed excretory passage of the organ in the event of respiration, i.e. inspiration or expiration.

When the lesion area is completely cured with the lapse of time after the administration of the stent, it is necessary to remove the biodegradable double stent 24 from the administered position.

The biodegradable stent 23 made of biodegradable polymer is gradually degraded away by bodily fluids while a lesion area is being cured.

When the biodegradable stent 23, which is made of biodegradable polymer and firmly maintains the biodegradable double stent 24 itself at the administered position by supporting the inner wall of the excretory passage of the organ, is degraded away, the primary stent 10 of the biodegradable double stent 24 becomes free from the administered position.

This free primary stent 10 has to be removed using separate tools such as surgical tools.

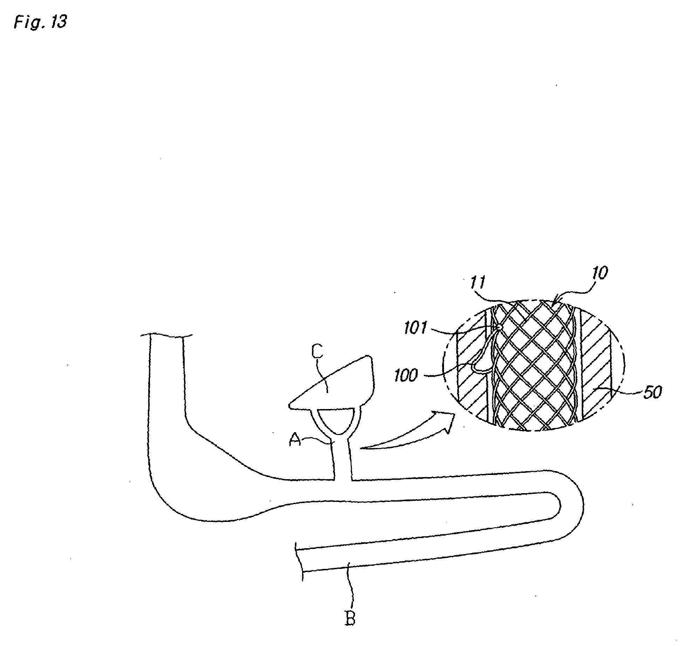

Similarly, as illustrated in FIG. 13, the hook-shaped stent 100 prevents the primary stent 10 from easily sliding due the external force, because the hook-shaped stent 100 also pressurizes the inner wall of the excretory passage of the organ after administration. As the primary stent 10 attempts to move downwards due to strong external force, the hook-shaped stent 100 still more strongly pressurizes the inner wall of the excretory passage of the organ in an outward direction. As a result, the primary stent 10 is prevented from deviating from the administered position.

At this time, similar to the biodegradable stent 23 made of biodegradable polymer, the hook-shaped stent 100 is also gradually degraded away by bodily fluids while the lesion area is being cured.

When the hook-shaped stent 100, which firmly maintains the biodegradable double stent 24 itself at the administered position by supporting the inner wall of the excretory passage of the organ, is degraded away, the primary stent 10 of the biodegradable double stent 24 becomes free from the administered position.

This free primary stent 10 has to be removed using separate tools such as surgical tools.

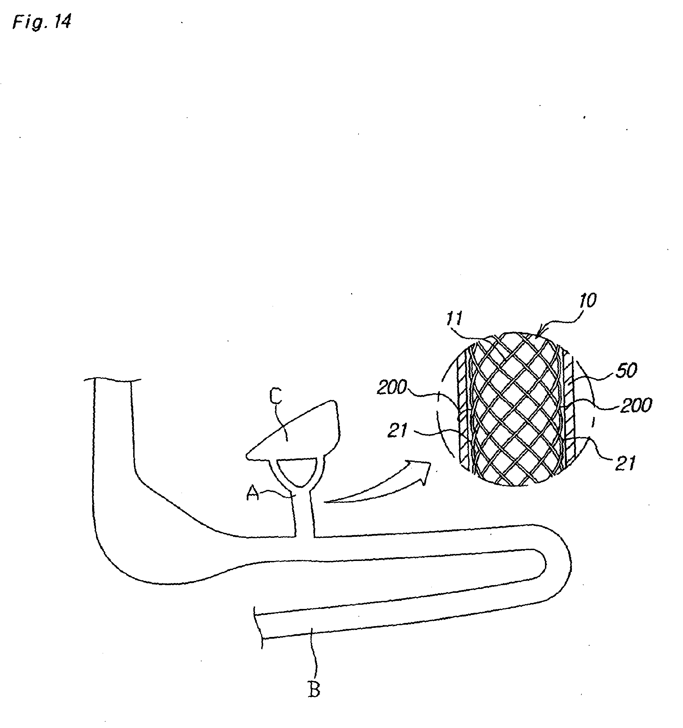

Further, as illustrated in FIG. 14, when the pressurization end 200 of the biodegradable stent 23 protruding outwards from the primary stent 10 is administered, the pressurization end 200 of the biodegradable stent 23 pressurizes the inner wall of the excretory passage of the organ.

This configuration prevents the primary stent 10 from sliding downwards due to the application of an external force. Further, since the biodegradable stent 23 is made of biodegradable polymer, the biodegradable stent 23 is gradually degraded away by bodily fluids while the lesion area is being cured. Thus, the primary stent 10 of the biodegradable double stent 24 becomes free from the administered position.

This free primary stent 10 has to be removed using separate tools such as surgical tools.

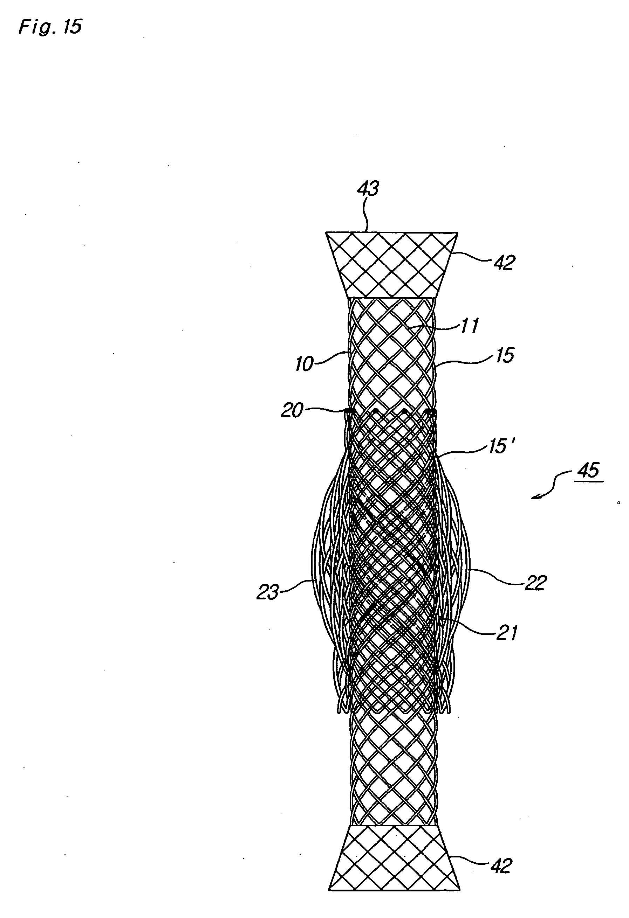

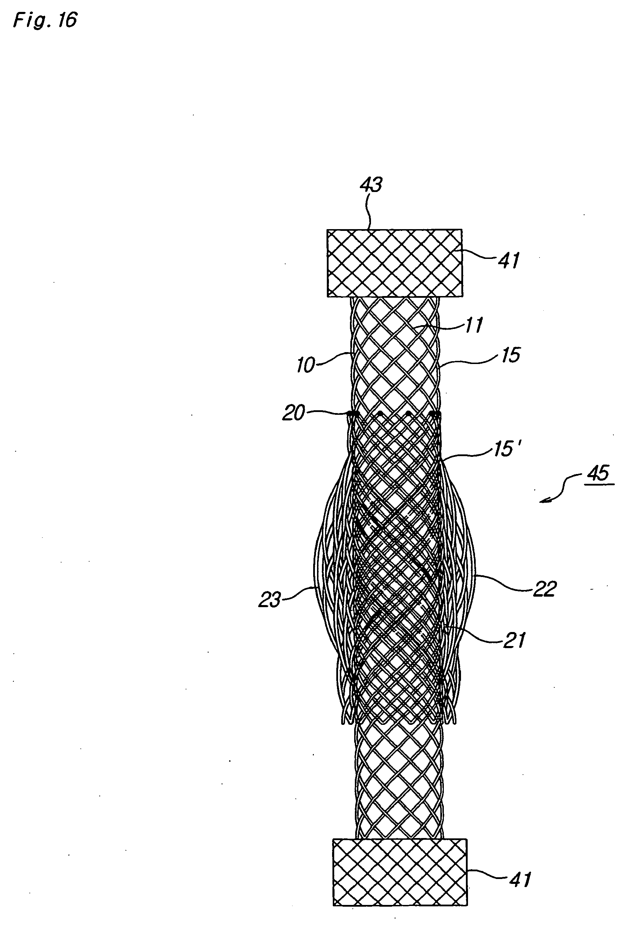

According to an embodiment of the present invention, the biodegradable double stent 24 as described above can be applied to a trumpet-shaped stent 45 having expanded portions 43, which are greater than the diameter of the cylindrical body 15 of the primary stent 10 at the opposite ends of the primary stent 10. For example, this trumpet-shaped stent 45 includes one having the expanded portions 43, each of which has a taper face 42 inclined at a predetermined angle as illustrated in FIG. 15 or each of which has a flat face 41 formed in a step shape as illustrated in FIG. 16.

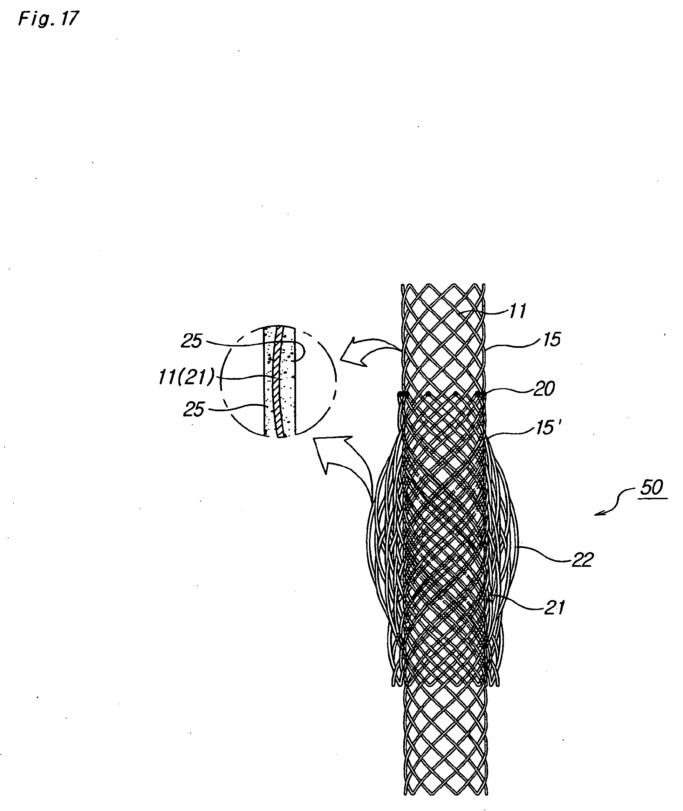

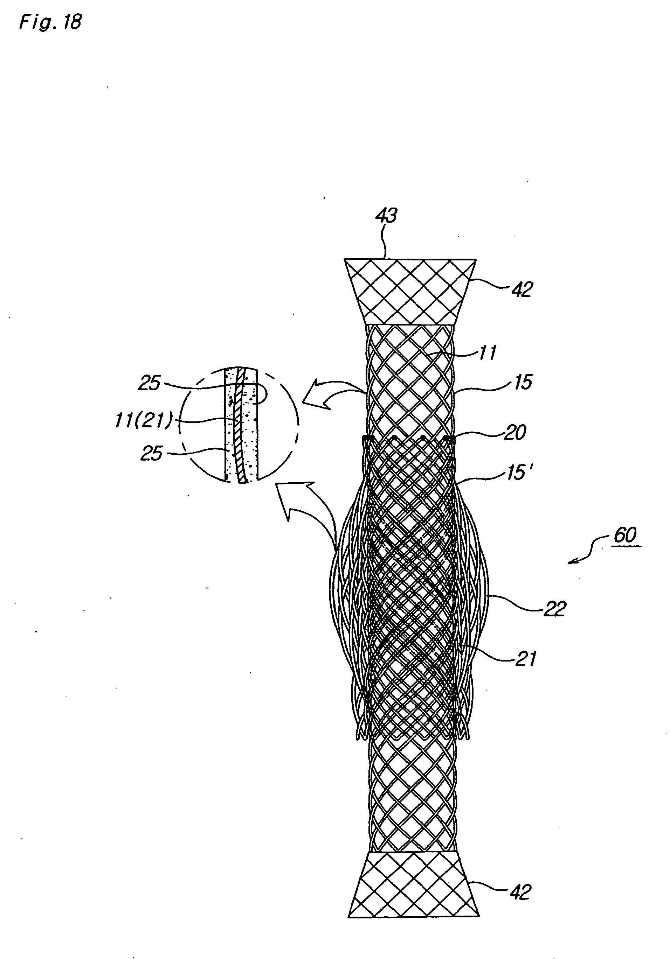

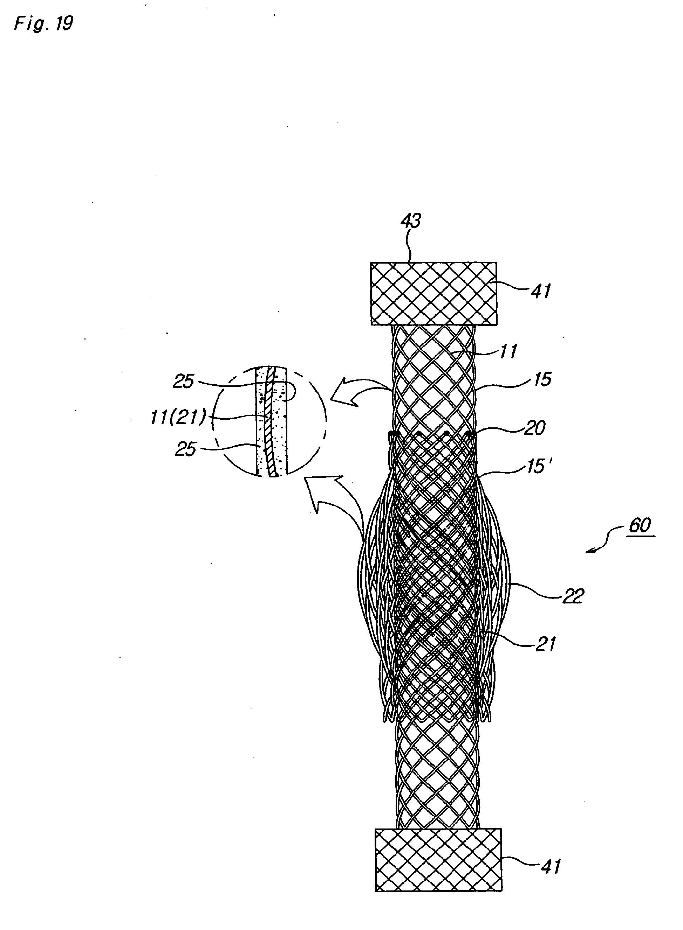

Further, as illustrated in FIGS. 17, 18 and 19, the biodegradable double stent 24 as described above can be applied to a thin-film-coated stent 50 or a thin-film-coated trumpet-shaped stent 60 in which a thin film 25 made of polytetrafluoroethylene (PTFE) or silicon can be formed on an inner and/or outer circumference of the primary stent 10 or the trumpet-shaped stent 45. This thin-film-coated stent 50 or the thin-film-coated trumpet-shaped stent 60 is used for preventing the lesion area from penetrating into the primary stent 10 while the aforementioned lesion area grows.

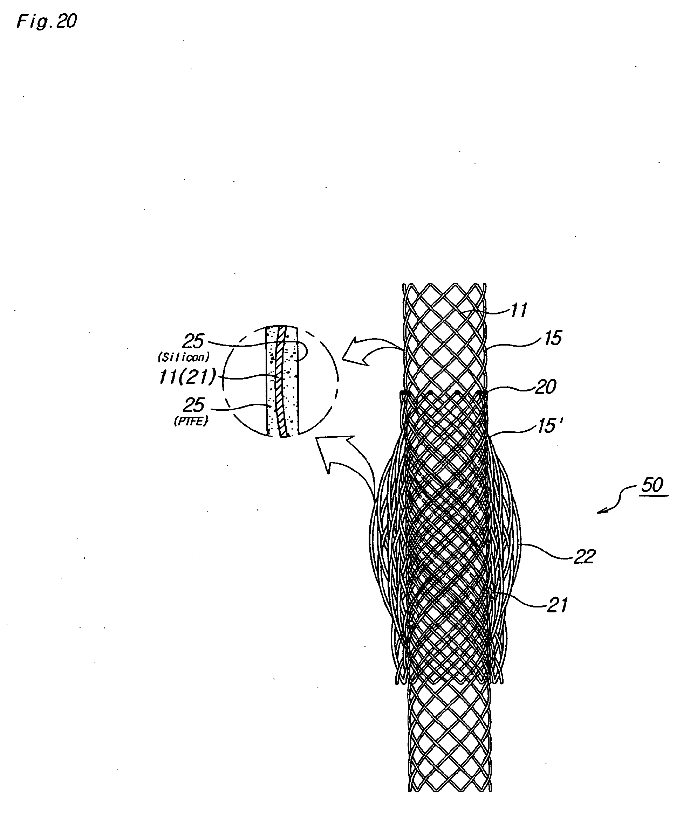

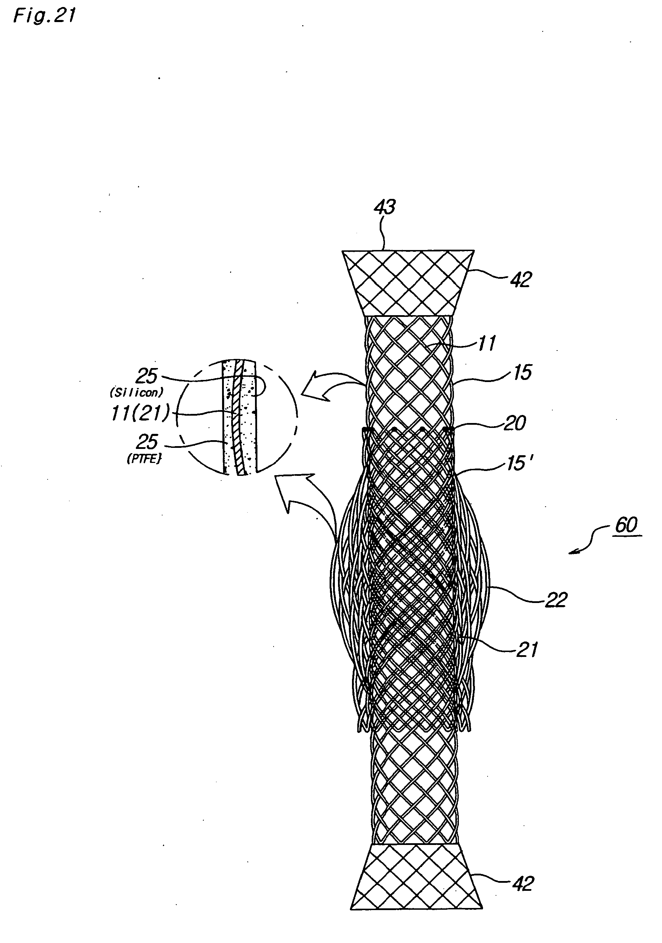

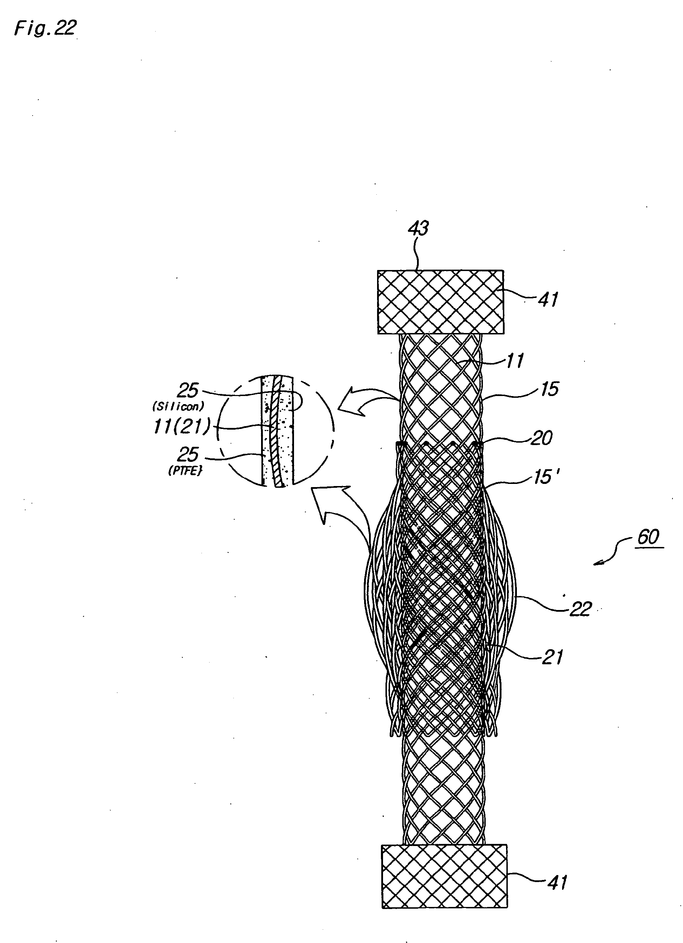

In this case, as illustrated in FIGS. 20, 21 and 22, if necessary, in order to maintain the biodegradable double stent at the lesion area for a long time after the biodegradable double stent has been administered, the inner circumference of the primary stent 10 or the trumpet-shaped stent 45 is selectively coated with PETE or silicon, while the outer circumference of the primary stent 10 or the trumpet-shape stent 45 is coated with a material, which is different from that coated on the inner circumference of the primary stent 10 or the trumpet-shaped stent 45. For example, if the inner circumference of the primary stent 10 or the trumpet-shaped stent 45 is coated with PETE, the outer circumference of the primary stent 10 or the trumpet-shaped stent 45 is coated with silicon.

In the latter case, since the inner and outer circumferences of the primary stent 10 or the trumpet-shaped stent 45 are coated with the different materials, the time required for degradation of the biodegradable stent 23 by bodily fluids is prolonged, as compared to the former case in which one of the inner and outer circumferences of the primary stent 10 or the trumpet-shaped stent 45 is coated with PETE or silicon. As such, the biodegradable double stent is maintained in the human body in proportion to the prolonged time, so that it can secure the excretory passage of the organ through outward pushing of the lesion area.

Although an exemplary embodiment of the present invention has been described for illustrative purposes, those skilled in the art will appreciate that various modifications, additions and substitutions are possible, without departing from the scope of the invention as disclosed in the accompanying claims.

## Claims

1. A double-tube type biliary stent comprising:
a primary stent (10) having a hollow cylindrical body (15) that has a plurality of rhombic spaces (12) formed by weaving an alloy wire (11) made of superelastic shape memory alloy or stainless steel so as to be criss-crossed and that is inserted into and expands an excretory passage of an organ; and
a biodegradable stent (23) having a hollow cylindrical body (15') that has a plurality of rhombic spaces (12') formed by weaving a separate wire (21) made of biodegradable polymer so as to be criss-crossed;
wherein the biodegradable stent (23) is located on an outer circumference of the primary stent (10), and is connected to the primary stent at one of opposite ends thereof;
**characterized in that** the biodegradable stent has a curved portion (28) curved outwards at an intermediate portion thereof.

2. The biodegradable double stent as set forth in claim 1, wherein the biodegradable polymer of the biodegradable stent (23) includes at least one selected from poly L-lactic acid (PLLA), poly lactic acid (PLA), polyglycolic acid (PGA), poly glycolide-co-L-lactide acid (PGLA), polydioxanone (PDO), and poly glycolide-co-caprolactone (PGCL).

3. The biodegradable double stent as set forth in claim 1, wherein the biodegradable stent (23) is located within an intermediate portion of the outer circumference of the primary stent (10) when coupled to the primary stent.

4. The biodegradable double stent as set forth in claim 1, wherein the biodegradable stent (23) is located beyond an intermediate portion of the outer circumference of the primary stent (10) when coupled to the primary stent.

5. The biodegradable double stent as set forth in claim 1, wherein the biodegradable stent (23) includes at least one hook-shaped stent (100), which is curved in a downward direction and is fastened to the primary stent (10) at opposite ends thereof using a fasting means.

6. The biodegradable double stent as set forth in claim 5, wherein the hook-shaped stents (100) are installed on the outer circumference of the primary stent (10) so as to be opposite to each other in a diametrical direction.

7. The biodegradable double stent as set forth in claim 5, wherein the hook-shaped stents (100) are installed on the outer circumference of the primary stent (10) in a longitudinal direction of the primary stent.

8. The biodegradable double stent as set forth in claim 5, wherein the hook-shaped stents (100) are installed on the outer circumference of the primary stent (10) at regular intervals in a longitudinal direction of the primary stent.

9. The biodegradable double stent as set forth in claim 5, wherein the hook-shaped stents (100) are installed on the outer circumference of the primary stent (10) in a zigzag shape in a longitudinal direction of the primary stent.

10. The biodegradable double stent as set forth in claim 1 or 5, wherein the biodegradable stent (23) is configured so that one end thereof is formed as a pressurization end (200) protruding outwardly from the outer circumference of the primary stent (10) and that remaining portions thereof are interwoven with the primary stent.

11. The biodegradable double stent as set forth in claim 10, wherein the pressurization end (200) includes one selected from a linear shape and a hook shape.

12. The biodegradable double stent as set forth in claim 1, wherein the primary stent (10) is coated with one of polytetrafluoroethylene and silicon on one of inner and outer circumferences thereof.

13. The biodegradable double stent as set forth in claim 1, wherein the primary stent (10) includes a trumpet-shaped stent (45), opposite ends of which are expanded.

14. The biodegradable double stent as set forth in claim 13, wherein the trumpet-shaped stent (45) is coated with one of polytetrafluoroethylene and silicon on one of inner and outer circumferences thereof.

15. The biodegradable double stent as set forth in claim 1, wherein the primary stent (10) is coated with one of polytetrafluoroethylene and silicon on inner and outer circumferences thereof so as to have different thin films.

16. The biodegradable double stent as set forth in claim 13, wherein the trumpet-shaped stent (45) is coated with one of polytetrafluoroethylene and silicon on inner and outer circumferences thereof so as to have different thin films.

## Patentansprüche

1. Doppelrohrgallenstent, umfassend
einen Primärstent (10) mit einem zylindrischen Hohlkörper (15), der eine Anzahl rhombische Räume (12) aufweist, die gebildet werden durch Weben eines Legierungsdrahts (11) aus einer superelastischen Formgedächtnislegierung oder Edelstahl, so dass er kreuzgewickelt wird und der in einen Ausführungsgang eines Organs eingeführt wird und diesen erweitert; und
einen biologisch abbaubaren Stent (23) mit einem zylindrischen Hohlkörper (15'), der eine Anzahl rhombische Räume (12') aufweist, die gebildet werden durch Weben eines gesonderten Drahts (21) aus einem biologisch abbaubaren Polymer, so dass er kreuzgewickelt wird;
worin der biologisch abbaubare Stent (23) sich auf einem äußeren Umfang des Primärstents (10) befindet, und mit dem Primärstent auf einem seiner gegenüber liegendenen Enden verbunden ist;
**dadurch gekennzeichnet, dass** der biologisch abbaubare Stent einen gewölbten Teil (28) hat, der an einem Mittenteil davon nach außen gewölbt ist.

2. Biologisch abbaubarer Doppelrohrstent gemäß Anspruch 1, worin das biologisch abbaubare Polymer des biologisch abbaubaren Stents (23) mindestens eines enthält aus Poly-L-Milchsäure (PLLA), Polymilchsäure (PLA), Polyglycolsäure (PGA), Polyglycolid-co-L-Milchsäure (PGLA), Polydioxanon (PDO) und Polyglycolid-co-caprolacton (PGCL).

3. Biologisch abbaubarer Doppelrohrstent gemäß Anspruch 1, worin der biologisch abbaubare Stent (23) sich in einem Mittenteil des äußeren Umfangs des Primärstents (10) befindet, wenn er mit dem Primärstent gekoppelt ist.

4. Biologisch abbaubarer Doppelrohrstent gemäß Anspruch 1, worin der biologisch abbaubare Stent (23) sich hinter einem Mittenteil des äußeren Umfangs des Primärstents (10) befindet, wenn er mit dem Primärstent gekoppelt ist.

5. Biologisch abbaubarer Doppelrohrstent gemäß Anspruch 1, worin der biologisch abbaubare Stent (23) mindestens einen hakenförmigen Stent (100) enthält, der nach unten gewölbt ist und mit einem Befestigungsmittel am Primärstent (10) an gegenüber liegenden Enden davon befestigt ist.

6. Biologisch abbaubarer Doppelrohrstent gemäß Anspruch 5, worin die hakenförmigen Stents (100) auf dem äußeren Umfang des Primärstents (10) installiert sind, so dass sie sich in einer diametralen Richtung gegenüber liegen.

7. Biologisch abbaubarer Doppelrohrstent gemäß Anspruch 5, worin die hakenförmigen Stents (100) auf dem äußeren Umfang des Primärstents (10) in einer Längsrichtung des Primärstents installiert sind.

8. Biologisch abbaubarer Doppelrohrstent gemäß Anspruch 5, worin die hakenförmigen Stents (100) in regelmäßigen Abständen auf dem äußeren Umfang des Primärstents (10) in einer Längsrichtung des Primärstents installiert sind.

9. Biologisch abbaubarer Doppelrohrstent gemäß Anspruch 5, worin die hakenförmigen Stents (100) in einer Zick-Zack-Form auf dem äußeren Umfang des Primärstents (10) in einer Längsrichtung des Primärstents installiert sind.

10. Biologisch abbaubarer Doppelrohrstent gemäß Anspruch 1 oder 5, worin der biologisch abbaubare Stent (23) so ausgestaltet ist, dass eines seiner Enden als Druckende (200) ausgebildet ist, das nach außen aus dem äußeren Durchmesser des Primärstents (10) hervorragt und so, dass seine übrigen Teile mit dem Primärstent verflochten sind.

11. Biologisch abbaubarer Doppelrohrstent gemäß Anspruch 10, worin das Druckende (200) eine Form enthält, ausgewählt aus einer linearen Form und einer Hakenform.

12. Biologisch abbaubarer Doppelrohrstent gemäß Anspruch 1, worin der Primärstent (10) mit einem aus Polytetrafluoroethylen und Silicium auf einem aus seinem inneren und seinem äußeren Umfang beschichtet ist.

13. Biologisch abbaubarer Doppelrohrstent gemäß Anspruch 1, worin der Primärstent (10) einen trompetenförmigen Stent (45) enthält, dessen gegenüber liegende Enden erweitert sind.

14. Biologisch abbaubarer Doppelrohrstent gemäß Anspruch 13, worin der trompetenförmige Stent (45) mit einem aus Polytetrafluoroethylen und Silicium auf einem aus seinem inneren und seinem äußeren Umfang beschichtet ist.

15. Biologisch abbaubarer Doppelrohrstent gemäß Anspruch 1, worin der Primärstent (10) mit einem aus Polytetrafluoroethylen und Silicium auf seinem inneren und seinem äußeren Umfang beschichtet ist, so dass diese unterschiedliche Dünnschichten aufweisen.

16. Biologisch abbaubarer Doppelrohrstent gemäß Anspruch 13, worin der trompetenförmige Stent (45) mit einem aus Polytetrafluoroethylen und Silicium auf seinem inneren und seinem äußeren Umfang beschichtet ist, so dass diese unterschiedliche Dünnschichten aufweisen.

## Revendications

1. Endoprothèse biliaire de type à double tube, comprenant :
une endoprothèse (10) primaire ayant un corps (15) cylindrique creux, qui a une pluralité d'espaces (12) en losange formés en tissant un fil (11) en un alliage superélastique à mémoire de forme ou en acier inoxydable, de manière à ce qu'il soit entrecroisé et en ce qu'elle est insérée dans un passage excrétoire d'un organe et s'y détend ; et
une endoprothèse (23) biodégradable ayant un corps (15') cylindrique creux, qui a une pluralité d'espaces (12') en losange formés en tissant un fil (21) distinct en polymère biodégradable, de manière à ce qu'il soit entrecroisé ;
dans laquelle l'endoprothèse (23) biodégradable est placée sur une circonférence extérieure de l'endoprothèse (10) primaire et est reliée à l'endoprothèse primaire à l'une de ses extrémités opposées ;
**caractérisée en ce que** l'endoprothèse biodégradable a une partie (28) courbée, courbée vers l'extérieur à une partie intermédiaire de celle-ci.

2. Endoprothèse double biodégradable suivant la revendication 1, dans laquelle le polymère biodégradable de l'endoprothèse (23) biodégradable comprend au moins l'un du poly(acide lactique L) (PLLA), du poly(acide lactique) (PLA), du poly(acide glycolique) (PGA), du poly glycolide-co-acide lactide L (PGLA), du polydioxanone (PDO) et du poly glycolide-co-caprolactone (PGCL).

3. Endoprothèse double biodégradable suivant la revendication 1, dans laquelle l'endoprothèse (23) biodégradable est placée dans une partie intermédiaire de la circonférence extérieure de l'endoprothèse (10) primaire lorsqu'elle est couplée à l'endoprothèse primaire.

4. Endoprothèse double biodégradable suivant la revendication 1, dans laquelle l'endoprothèse (23) biodégradable est placée au-delà d'une partie intermédiaire de la circonférence extérieure de l'endoprothèse (10) primaire lorsqu'elle est couplée à l'endoprothèse primaire.

5. Endoprothèse double biodégradable suivant la revendication 1, dans laquelle l'endoprothèse (23) biodégradable comprend au moins une endoprothèse (100) en forme de crochet, qui est courbée dans une direction vers le bas et est fixée à l'endoprothèse (10) primaire à ses extrémités opposées en utilisant un moyen de fixation.

6. Endoprothèse double biodégradable suivant la revendication 5, dans laquelle les endoprothèses (100) en forme de crochet sont installées sur la circonférence extérieure de l'endoprothèse (10) primaire, de manière à être opposées l'une à l'autre dans une direction diamétrale.

7. Endoprothèse double biodégradable suivant la revendication 5, dans laquelle les endoprothèses (100) en forme de crochet sont installées sur la circonférence extérieure de l'endoprothèse (10) primaire dans une direction longitudinale de l'endoprothèse primaire.

8. Endoprothèse double biodégradable suivant la revendication 5, dans laquelle les endoprothèses (100) en forme de crochet sont installées sur la circonférence extérieure de l'endoprothèse (10) primaire à des intervalles réguliers dans une direction longitudinale de l'endoprothèse primaire.

9. Endoprothèse double biodégradable suivant la revendication 5, dans laquelle les endoprothèses (100) en forme de crochet sont installées sur la circonférence extérieure de l'endoprothèse (10) primaire suivant une forme en zigzag dans une direction longitudinale de l'endoprothèses primaire.

10. Endoprothèse double biodégradable suivant la revendication 1 ou 5, dans laquelle l'endoprothèse (23) biodégradable est configurée de manière à ce que l'une de ses extrémités soit formée en tant qu'extrémité (200) de pressurisation faisant saillie vers l'extérieur de la circonférence extérieure de l'endoprothèse (10) primaire et à ce que ses parties restantes soient entrelacées avec l'endoprothèse primaire.

11. Endoprothèse double biodégradable suivant la revendication 10, dans laquelle l'extrémité (200) de pressurisation comprend l'une sélectionnée d'une forme linéaire et d'une forme en crochet.

12. Endoprothèse double biodégradable suivant la revendication 1, dans laquelle l'endoprothèse (10) primaire est revêtue de l'un du polytétrafluoroéthylène et d'une silicone sur l'une de ses circonférences intérieure et extérieure.

13. Endoprothèse double biodégradable suivant la revendication 1, dans laquelle l'endoprothèse (10) primaire comprend une endoprothèse (45) en forme de trompette, dont les extrémités opposées sont dilatées.

14. Endoprothèse double biodégradable suivant la revendication 13, dans laquelle l'endoprothèse (45) en forme de trompette est revêtue de l'un du polytétrafluoroéthylène et d'une silicone sur l'une de ses circonférences intérieure et extérieure.

15. Endoprothèse double biodégradable suivant la revendication 1, dans laquelle l'endoprothèse (10) primaire est revêtue de l'un du polytétrafluoroéthylène et d'une silicone sur ses circonférences intérieure et extérieure, de manière à avoir des pellicules minces différentes.

16. Endoprothèse double biodégradable suivant la revendication 13, dans laquelle l'endoprothèse (45) en forme de trompette est revêtue de l'un du polytétrafluoroéthylène et d'une silicone sur ces circonférences intérieure et extérieure, de manière à avoir des pellicules minces différentes.
